# EUROPÄISCHE PATENTSCHRIFT

(11) **EP 2 812 687 B1**
(45) Veröffentlichungstag und Bekanntmachung des Hinweises auf die Patenterteilung: **22.06.2016**
(21) Anmeldenummer: 13705911.9
(22) Anmeldetag: 07.02.2013
(51) Int. Cl.: G01N 33/487

(54) **ELEKTROPHYSIOLOGISCHE MESSANORDNUNG UND ELEKTROPHYSIOLOGISCHES MESSVERFAHREN**
ELECTROPHYSIOLOGICAL MEASURING ARRANGEMENT, AND ELECTROPHYSIOLOGICAL MEASURING METHOD
ENSEMBLE DE MESURE ÉLECTROPHYSIOLOGIQUE ET PROCÉDÉ DE MESURE ÉLECTROPHYSIOLOGIQUE

(30) Priorität: 08.02.2012 DE 102012002459
(43) Veröffentlichungstag der Anmeldung: 17.12.2014
(73) Patentinhaber: Universität Rostock, 18057 Rostock (DE)
(72) Erfinder: KLINK, Oliver, 18057 Rostock (DE); KÖSTER, Philipp Julian, 18059 Rostock (DE); TAUTORAT, Carsten, 18109 Rostock (DE); SCHEFFLER, Uwe, 18184 Broderstorf (DE)
(74) Vertreter: Müller Hoffmann & Partner
(86) Internationale Anmeldenummer: PCT/EP2013/000369
(87) Internationale Veröffentlichungsnummer: WO 2013/117337

(56) Entgegenhaltungen:
- US-A1- 2003 098 248
- KHOSHMANESH K ET AL: "Dielectrophoretic platforms for bio-microfluidic systems", BIOSENSORS AND BIOELECTRONICS, ELSEVIER BV, NL, Bd. 26, Nr. 5, 15. Januar 2011 (2011-01-15), Seiten 1800-1814, XP027580077, ISSN: 0956-5663 [gefunden am 2010-12-30]

## Beschreibung

### GEBIET DER ERFINDUNG

Die vorliegende Erfindung betrifft einen Träger für eine elektrophysiologische Messanordnung, eine elektrophysiologische Messanordnung sowie ein elektrophysiologisches Messverfahren.

### HINTERGRUND DER ERFINDUNG

Im Bereich der Elektrophysiologie werden verschiedene Verfahren und Einrichtungen eingesetzt, um biologische Objekte - insbesondere also Zellen im weitesten Sinne, Zellorganellen, Oozyten und deren Fragmente - im Hinblick auf in jeweilige Membranen integrierte und/oder dort angelagerte Proteine und deren Transporteigenschaften hin zu untersuchen, wobei auch Vesikel, Liposomen oder andere mehr oder weniger künstliche Systeme zum Einsatz kommen können. Dabei werden häufig zwischen einer Messelektrode und einer Gegenelektrode, welche zwischen dem biologischen Objekt angeordnet werden, elektrische Ströme und/oder elektrische Spannungen gemessen, die Aufschluss geben sollen über die zu Grunde liegenden physiologischen Prozesse, insbesondere Transportprozesse, Konformationsänderungen und dergleichen.

Aufgrund der häufig vorliegenden vergleichsweise sehr geringen Signalstärken sind zum Erzielen eines geeigneten Signal-zu-Rausch-Verhältnisses hohe Abdichtwiderstände, das heißt also möglichst geringe elektrische Restleitfähigkeiten, über die Membran selbst oder im Berührungs- oder Kontaktbereich zwischen der Membran des zu untersuchenden biologischen Objekts und der Aperturwand von Vorteil.

Bisher lassen sich bei bekannten elektrophysiologischen Messverfahren und Messanordnungen der Abdichtwiderstand oder die elektrische Restleitfähigkeit zwischen Zellinnerem und Zelläußerem - oder allgemeiner zwischen Membraninnenseite und Membranaußenseite - des zu vermessenden biologischen Objekts nicht in ausreichendem Maße steuern. Dies führt dazu, dass ganz allgemein die Abdichtwiderstände häufig zu gering sind, so dass sich ungünstige Signal-zu-Rausch-Verhältnisse einstellen. Oft ist aber auch gerade das Anlagern und das Ausbilden eines Abdichtwiderstands selbst insbesondere zeitlich schlecht steuerbar sind, so dass es zum Beispiel schon bei Vorbereitungen zu einer Messung zu zu diesem Zeitpunkt noch unerwünschten Anlagerungen in der Messanordnung kommen kann, die, wenn sie gelöst werden, zu einer Kontamination und somit zu einer Verschlechterung bei weiteren, dann gewünschten Anlagerungen führen können oder eine spätere Anlagerung gar verhindern.

Die US 2003/0098248 A1 beschreibt ein Analysesystem mit einer Mehrzahl von Aperturen zum Positionieren von Zellen und dergleichen, wobei die Aperturen in einem Substrat als Durchgangslöcher ausgebildet sind, das Substrat sich in einem wässrigen Messmedium befindet und oberhalb und unterhalb des Substrats Mess- und Gegenelektroden ausgebildet sind. Durch Anordnen der Zellen im Bereich einer jeweiligen Apertur wird beim Anlagern ein Abdichtwiderstand erzeugt. Über die Mess- und Gegenelektroden werden dann Strom- und/oder Spannung der zu vermessenden Zelle unter verschiedenen Bedingungen ermittelt.

Die Veröffentlichung "Dielectrophoretic platforms for bio-microfluidic systems" beschreibt eine Anordnung, mit deren Hilfe über dielektrophoretische Prozesse zu vermessenden Proben und deren Bestandteile bewegt und räumlich positioniert werden können.

### ZUSAMMENFASSUNG DER ERFINDUNG

Der Erfindung liegt die Aufgabe zu Grunde, einen Träger für eine elektrophysiologische Messanordnung, eine elektrophysiologische Messanordnung sowie ein elektrophysiologisches Messverfahren zu schaffen, bei welchen es möglich ist, das Anlagern eines zu vermessenden biologischen Objekts sowie das Ausbilden eines Abdichtwiderstands bei der Anlagerung zwischen dem zu vermessenden biologischen Objekt und dem Messsystem möglichst zuverlässig zu steuern.

Die der Erfindung zu Grunde liegende Aufgabe wird bei einem Träger für eine elektrophysiologische Messanordnung erfindungsgemäß mit den Merkmalen des unabhängigen Patentanspruchs 1 und bei einer elektrophysiologischen Messanordnung erfindungsgemäß mit den Merkmalen des unabhängigen Patentanspruchs 9 gelöst. Des Weiteren wird die der Erfindung zu Grunde liegende Aufgabe bei einem elektrophysiologischen Messverfahren erfindungsgemäß mit den Merkmalen des unabhängigen Patentanspruchs 10 gelöst. Vorteilhafte Weiterbildungen sind in den abhängigen Ansprüchen beschrieben.

Die vorliegende Erfindung schafft zum einen eine elektrophysiologische Messanordnung mit einem Aperturbereich - also mit einem Bereich, der mindestens eine Apertur oder Messapertur aufweist oder bildet - zum gesteuerten abdichtenden oder sealenden Anlagern eines biologischen Objekts, z.B. einer Zelle, einer Zellorganelle, eines Vesikels, eines Liposoms, einer natürlichen oder künstlichen Membran, z.B. einer Lipiddoppelschicht, oder dergleichen oder eines Fragments davon. Dabei ist der Aperturbereich mit mindestens einer Apertur ausgebildet sowie mit einem Wandbereich, welcher die Apertur bildend umgibt. Der Wandbereich weist eingebettet in seinem Inneren eine Steuerelektrodenanordnung auf. Die Steuerelektrodenanordnung ist derart steuerbar mit einem elektrischen Potential beaufschlagbar ist, dass dadurch zumindest eine Innenwand des Wandbereichs, welche der Apertur zugewandt ist, steuerbar derart mit einer Oberflächenladung ausbildbar ist, dass dadurch das abdichtende oder sealende Anlagern eines biologischen Objekts am Aperturbereich steuerbar ist.

Zusätzlich kann die direkte oder indirekte Beeinflussung von Molekülen in Wandnähe durch elektrische Felder erfolgen, sowie z.B. auch durch Erzeugen von Ringströmen, z.B. auch mittels Induktion, d.h. elektromagnetisch.

Es ist somit eine Kernidee der vorliegenden Erfindung, bei einer elektrophysiologischen Messanordnung mit einem Aperturbereich, welcher zum abdichtenden oder sealenden Anlagern, also zum Anlagern mit hohem Abdichtwiderstand in Bezug auf ein zu untersuchendes biologisches Objekt ausgebildet ist, in einem Wandbereich, welcher eine Apertur eines Aperturbereichs bildet und umgibt, in dessen Innerem eingebettet eine Steuerelektrodenanordnung vorzusehen. Diese Steuerelektrodenanordnung ist gegenüber der sonstigen Messanordnung - insbesondere gegenüber dem biologischen Objekt, einem Elektrolytbad, in dem dieses sich befindet, und etwaigen Mess- und Gegenelektroden gegenüber, elektrisch isoliert und kann steuerbar mit einem elektrischen Potential beaufschlagt werden, um dadurch zumindest an oder in der Innenwand des Wandbereichs, also zur Apertur zugewandt, in gesteuerter Art und Weise eine Oberflächenladung auszubilden.

Das steuernde Ausbilden der Oberflächenladung führt dann über eine entsprechende elektrische Wechselwirkung dazu, dass auch das abdichtende oder sealende Anlagern eines zu untersuchenden biologischen Objekts gesteuert werden kann, nämlich dadurch, dass entweder eine abstoßende Wechselwirkung provoziert wird, die ein ungewolltes Anlagern und damit eine zu vermeidende Kontamination verhindert, oder aber über eine anziehende Wechselwirkung eine Annäherung, ein Anlagern und ein Abdichten im Sinne eines Seals in Bezug auf das zu untersuchende biologische Objekt fördert.

Es ist somit erfindungsgemäß möglich, in einer Vorbereitungsphase zu einer Messung eine Anlagerung und einen Seal zu verhindern, um dann in der eigentlichen Messphase - oder unmittelbar davor - das gewünschte biologische Objekt zur Anlagerung und zur Ausbildung eines Seals mit der Apertur und dem Wandbereich der Apertur zu zwingen und die Stärke des Seals im Sinne eines erhöhten Abdichtwiderstands oder einer stark erniedrigten Restleitfähigkeit sowie einer verstärkten mechanischen Stabilität des Seals zu verbessern.

Als biologische Objekte, die einer Untersuchung zuzuführen sind, können dabei Zellen, Zellorganellen, Oozyten, Bakterien oder deren Kombinationen oder Fragmente, jeweils im weitesten Sinne, verwendet werden. Auch sind künstliche oder teilweise künstliche im Wesentlichen biologische Strukturen denkbar, zum Beispiel in Form von Vesikeln, Liposomen, Mizellen, Membranfragmente oder dergleichen, in welche in natürlicher oder künstlicher Art und Weise Proteine ein- und/oder angelagert sind.

Die zu untersuchenden Objekte können ganz allgemein natürliche oder teilweise oder vollständig künstliche biologische Objekte sein. Auch sind nicht-biologische Objekte untersuchbar, um z.B. reine Lipidstrukturen und deren Modifikationen zu untersuchen. Im Folgenden wird ausschließlich von biologischen Objekten gesprochen, wobei jedoch sämtliche, in diesem Sinne beschriebenen Variationen als Messobjekte mit umfasst sein sollen.

Erfindungsgemäß wird mit anderen Worten erreicht, dass einerseits in der Vorbereitungsphase Verunreinigungen des Anlagerungsbereichs, nämlich der Wände der Apertur verhindert werden. Andererseits können ausgewählte zu untersuchende biologische Objekte mit einem verbesserten Abdichtwiderstand angelagert und danach vermessen werden, so dass sich ein besseres Signal-zu-RauschVerhältnis einstellt und die Anlagerung mechanisch stabilisiert wird, z.B. mit der Folge einer verlängerten Messzeitspanne und einer verbesserten Zuverlässigkeit in Bezug auf die Messergebnisse. Zusätzlich ergibt sich erfindungsgemäß auch die Möglichkeit, eine ggf. bereits erfolgte Verunreinigung des Anlagerungsbereichs, also z.B. der Aperturwand, durch Applikation einer entsprechend gewählten Gleich- oder Wechselspannung mittels der Steuerelektrodenanordnung zu vermindern oder gar zu entfernen.

Durch die Wahl der Polarität des der Steuerelektrodenanordnung aufgeprägten elektrischen Potentials wird entsprechend die Polarität der Oberflächenladung an der Wandinnenseite oder Innenwand des Wandbereichs der Apertur beeinflusst. Dadurch können Art und Stärke die Wechselwirkung in Abhängigkeit von der Ladung der Membran der biologischen Objekte auf deren Außen- und Innenseite beeinflusst werden.

Bei einer Ausführungsform der erfindungsgemäßen Messanordnung ist der Aperturbereich im Bereich eines Trägers, der eine Oberseite und eine Unterseite aufweist, ausgebildet. Dabei kann dann ein jeweiliger eine Apertur bildender Wandbereich in Bezug auf die Oberseite und/oder in Bezug auf die Unterseite des Trägers teilweise oder vollständig hervorstehen. Der vorzusehende Träger kann auch als Basis, Substrat oder Grundsubstrat bezeichnet werden. Das Vorsehen eines derartigen Substrats oder eines derartigen Trägers stabilisiert die Messanordnung und insbesondere die Anordnung des angeordneten zu untersuchenden biologischen Objekts als solche mechanisch und ermöglicht eine makroskopische Unterteilung der Messanordnung in Bezug auf das der Messung zu Grund zu legende Elektrolytbad im Sinne der Unterteilung einer Messküvette oder Nasszelle in Kompartimente mit Mess- und Gegenelektrode.

Ein jeweiliger eine Apertur bildender Wandbereich kann auch in der Innenwand des Loches in Kombination mit der Elektrodenanordnung integriert ausgebildet sein.

Auf der Grundlage des Substrats oder des Trägers können dann ein oder mehrere Aperturen mit entsprechenden Wandbereichen in Bezug auf die Oberseite herausragend oder sich hervorhebend ausgebildet werden. Alternativ dazu können diese auch an der Oberseite planar abschließend nach innen ausgestülpt sein, um auf der Unterseite des Trägers oder des Substrats hervorzustehen; dies ist jedoch nicht zwingend und kann bei entsprechender Dicke der Membran entfallen. Das Ausmaß des jeweiligen Einstülpens oder Hervortretens beeinflusst die Innenwand des jeweiligen Wandbereichs und damit die zur Verfügung stehende Wechselwirkungsfläche mit der Membran des biologischen Objekts. Die Wahl des Ausmaßes des Hervorragens oder Einstülpens ermöglicht darüber hinaus eine Anpassung an die jeweilig zur Verfügung stehenden Messobjekte, zum Beispiel im Hinblick auf deren Form oder Anzahl in der Messlösung.

Der Träger kann als - insbesondere planares - Plattenelement mit Vorder- oder Oberseite und mit Rück- oder Unterseite ausgebildet sein. Auch andere Geometrien sind denkbar.

Alternativ dazu kann von der Plattenform abgewichen werden, in dem z.B. die Form einer Pipette, z.B. im Sinne einer klassischen Patchpipette aufgegriffen wird.

Ein eine Apertur bildender Wandbereich kann nach Art einer Mantelfläche oder als eine Kombination von Mantelflächen ausgebildet sein. Dabei kann jeweils auf den Mantel eines Zylinders, eines Prismas, eines Kegelstumpfs und/oder eines Pyramidenstumpfs zurück gegriffen werden, mit entsprechender Wandstärke. Im Hinblick auf die Form des Wandbereichs zur Ausbildung der Apertur stehen also vielfältige Möglichkeiten zur Verfügung. Diese können ausgewählt werden in Abhängigkeit von der Form und den weiteren - z.B. mechanischen, geometrischen und/oder elektrischen - Eigenschaften der zu untersuchenden biologischen Objekte.

Alternativ dazu kann der eine Apertur bildende Wandbereich von einem Rand oder Randbereich gebildet werden, so dass die Apertur quasi als planares Loch in dem zu Grunde liegenden Substrat ausgebildet ist und dabei die Steuerelektrodenanordnung in den Randbereich des planaren Lochs eingebettet ist und diesen mit einer entsprechenden Oberflächenladung beaufschlagt, um das Anlagern und Sealen fördernd oder hemmend zu beeinflussen.

Ein eine Apertur bildender Wandbereich kann aus einem Material aus der Gruppe von Materialien ausgebildet sein, welche Glas, Quarzglas, Silizium, Kohlenstoff und deren Kombinationen aufweist. Auch in Bezug auf die Materialwahl können die Eigenschaften der zu Grunde liegenden biologischen Objekte berücksichtigt werden, zum Beispiel im Hinblick auf die Oberflächenstruktur oder Oberflächenladung der Membranaußenseite und/oder der Membraninnenseite der biologischen Objekte, zum Beispiel auch, um ein besonders inniges Anhaften und damit die Steigerung des Abdichtwiderstands beim Seal weiter zu unterstützen.

Die Steuerelektrodenanordnung kann ein oder mehrere und im eine Apertur bildenden Wandbereich integrierte oder eingebettete Elektrodenelemente aufweisen. Diese können beliebige Formen aufweisen, insbesondere in Form von Ringen, Streifen oder dergleichen. Hier sind jedoch sämtliche Formen für die Elektrodenelemente der Steuerelektrodenanordnung denkbar, solange nur gewährleistet ist, dass eine elektrische Isolation gegenüber dem Elektrolytbad, dem zu untersuchenden biologischen Objekt und insbesondere gegenüber den Elektroden der Messelektrodenanordnung und der Gegenelektrodenanordnung gewährleistet ist. Auch kann die Form und Anzahl der Elektrodenelemente vom Aufbau, der Form und der Struktur der Apertur und des Wandbereichs abhängig gemacht werden. Dies kann auch zur besseren Steuerbarkeit und insbesondere zur Intensivierung der Wechselwirkung des biologischen Objekts mit dem Wandbereich der Apertur vorteilhaft eingesetzt werden.

Die Elektroden können auch einen gewissen Abstand von der Wandfläche aufweisen, und zwar in Abhängigkeit des zu erzeugenden elektrischen Feldes und der einfachen Integrierbarkeit der Elektroden.

Die Steuerelektrodenanordnung und insbesondere deren Elektrodenelemente können in Bezug auf die Wandstärke eines oder des die Apertur bildenden Wandbereichs asymmetrisch ausgebildet und insbesondere näher zur Innenwand, welcher der Apertur zugewandt ist, hin angeordnet sein. Auch dies kann der Anpassung der Geometrie des Potentials und damit der Wechselwirkung zwischen dem biologischen Objekt und der Apertur und dessen Wandbereich dienen.

Die Steuerelektrodenanordnung und insbesondere deren Elektrodenelemente können aus einem Material aus der Gruppe von Materialien ausgebildet sein, die metallische Materialien, Gold, Tantal, Platin, Gold-Tantal-Platin, dotiertes, insbesondere hochdotiertes Polysilizium, Indiumzinnoxid, elektrisch leitfähige organische Materialien aufweist. Grundsätzlich sind jedoch sämtliche elektrisch leitfähigen oder teilweise elektrisch leitfähigen Materialien denkbar, die die Übertragung eines elektrischen Potentials ermöglichen, wobei darüber hinaus auch herstellungstechnische Aspekte berücksichtigt werden können. Insbesondere kann auch daran gedacht werden, leitfähige organische Materialien, dotierte Halbleitermaterialien oder dergleichen zu verwenden, solange dem keine prozesstechnischen Hindernisse entgegenstehen, zum Beispiel im Hinblick auf eine Stabilität der einzusetzenden Materialen bei hohen Prozesstemperaturen oder dergleichen.

Der Durchmesser der Apertur und insbesondere der Innendurchmesser eines oder des die Apertur bildenden Wandbereichs können einen Wert im Bereich von etwa 0 *µ*m bis etwa 50 *µ*m, vorzugsweise im Bereich im Bereich von etwa 1 *µ*m bis etwa 50 *µ*m aufweisen. Ein oder der eine Apertur bildende Wandbereich kann über der Oberseite oder der Unterseite des Trägers oder des Substrats eine Höhe oder Tiefe im Bereich von etwa 0 *µ*m bis etwa 20 *µ*m aufweisen.

Die Angaben derartiger Maße sei hier nicht beschränkend. Vielmehr orientieren sich die Maße in Bezug auf Höhe und Tiefe der Wandbereiche und deren Durchmesser an den geometrischen Gegebenheiten der zu untersuchenden biologischen Objekte, insbesondere also an deren Größe und an den mechanischen Eigenschaften ihrer Membranen.

Zur Ausbildung eines Messkreises können eine Messelektrode im Bereich oder innerhalb der Apertur oder in einem Bereich auf der Rück- oder Unterseite des Trägers oder Substrats vorgesehen sein.

Es kann eine Gegenelektrode außerhalb der Apertur und im Bereich auf der Vorder- oder Oberseite des Trägers vorgesehen sein.

Messelektrode und Gegenelektrode sind vorzugsweise auf gegenüberliegenden Seiten des Trägers oder Substrats oder der Messapertur angeordnet, jedenfalls so, dass bei Anlagerung eines zu vermessenden vorzugsweise biologischen Objekts dieses zwischen den Elektroden angeordnet ist und bei Ausbildung eines geeigneten Seals diese praktisch elektrisch trennt, vorzugsweise mit einem sehr hohen Widerstand, idealerweise größer als 1 GΩ.

Der grundlegende Aufbau für die erfindungsgemäße elektrophysiologische Messanordnung gemäß dieser Ausführungsform beinhaltet also insbesondere das Vorsehen einer Messelektrodenanordnung und einer Gegenelektrodenanordnung, zwischen welchen ein elektrischer Strom und/oder eine elektrische Spannung gemessen werden kann, wobei zwischen der Messelektrodenanordnung und der Gegenelektrodenanordnung gerade das zu vermessende biologische Objekt im Bereich der Apertur und deren Wandbereich derart angeordnet ist oder wird, dass durch die abdichtende oder sealende Anlagerung die Restleitfähigkeit, also die Leitfähigkeit zwischen der Membran des biologischen Objekts und dem Wandbereich der Apertur, möglichst gering ist, so dass die tatsächlich gemessenen elektrischen Ströme und/oder elektrischen Spannungen als von den Eigenschaften der Membran des biologischen Objekts hervorgerufen und angesehen werden können, zum Beispiel durch Transportprozesse, Ladungsverschiebungen innerhalb oder über die Membran, durch Substratbindung oder Abgabe oder dergleichen.

Gemäß einem weiterem Aspekt der vorliegenden Erfindung wird ein elektrophysiologisches Messverfahren geschaffen. Dieses wird insbesondere unter Verwendung einer elektrophysiologischen Messanordnung gemäß der vorliegenden Erfindung ausgeführt. Dabei wird zum Steuern eines abdichtenden oder sealenden Anlagerns eines zu vermessenden biologischen Objekts an einer Apertur eines Aperturbereichs die Innenwand eines die Apertur bildenden Wandbereichs gesteuert eine Oberflächenladung ausgebildet, insbesondere mit direkter oder indirekter Beeinflussung von Molekülen in der Nähe des Wandbereichs durch elektrische Felder, durch Erzeugen von Ringströmen und/oder mittels Induktion, d.h. elektromagnetisch, wobei insbesondere zum Verhindern einer abdichtenden oder sealenden Anlagerung eine negative Oberflächenladung und zum Unterstützen einer sealenden Anlagerung eine positive Oberflächenladung ausgebildet werden kann. Je nach den elektrischen Eigenschaften der anzulagernden Membran, müssen die Polaritäten ggf. umgekehrt gewählt werden. Es können durch geeignete Wechselspannungen auch Anlagerungen modifiziert werden, z.B. im Sinne einer Dielektrophorese.

Beim erfindungsgemäßen Messverfahren kann also auch eine direkte Beeinflussung von Molekülen in Wandnähe durch elektrische Felder zum Tragen kommen oder auch durch ein Induzieren von Ringströmen, z.B. auch induktiv, d.h. elektromagnetisch.

Entsprechend ist also ein Kernaspekt, welcher dem erfindungsgemäßen elektrophysiologischen Messverfahren zu Grunde liegt, das gesteuerte Beaufschlagen der Apertur und insbesondere deren Wandbereich mit einer Oberflächenladung, die über eine elektrische Wechselwirkung mit einem biologischen Objekt ein abdichtendes oder sealendes Anlagern im Sinne einer Unterstützung oder eines Verhinderns steuert. Dabei kann eine Feldbeeinflussung nicht nur direkt auf der Wandoberfläche, sondern Feldbeeinflussung auch in einem Abstand von der Wand zum Tragen kommen.

Bei dem erfindungsgemäßen Messverfahren und mit der erfindungsgemäßen Messanordnung können Messsignale insbesondere auch in kapazitiver Art und Weise gemessen werden, um z.B. Einzelkanalaktivitäten zu ermitteln.

Durch das Beaufschlagen der erfindungsgemäßen Steuerelektrodenanordnung können - z.B. im Sinne einer Elektroosmose - zu untersuchende biologisch Objekte, also z.B. Zellen, abgestoßen, eingezogen usw. werden.

Mit mehreren Steuerelektroden oder einem modifizierten Aufbau ist es erfindungsgemäß auch möglich, eine elektrische Kraft in ihrem Feldverlauf entsprechend zu modellieren, um die Kraft z.B. durch Wahl einer geeigneten Feldgeometrie zu fokussieren und/oder um ein Standardprotokoll zu realisieren.

Diese und weitere Aspekte der vorliegenden Erfindung werden auf der Grundlage der beigefügten Zeichnungen näher erläutert.

### KURZBESCHREIBUNG DER FIGUREN

- **Fig. 1A**: zeigt in geschnittener Seitenansicht eine erste Ausführungsform der erfindungsgemäßen elektrophysiologischen Messanordnung, bei welcher der Wandbereich einer Apertur sich über der Oberseite eines zu Grunde liegenden Trägers erhebt.
- **Fig. 1B - 1D**: zeigen in schematischer und geschnittener Draufsicht verschiedene Querschnittsformen einer Apertur und des jeweils zu Grunde liegenden Wandbereichs.
- **Fig. 2A, 2B**: zeigen in schematischer und geschnittener Seitenansicht Details der Ausbildung der Oberflächenladung am Wandbereich einer Apertur bei einer symmetrischen bzw. einer asymmetrischen Anordnung der Steuerelektrode im Wandbereich.
- **Fig. 3 - 7**: zeigen in analoger Art und Weise wie Fig. 1A in schematischer und geschnittener Seitenansicht Ausführungsformen für eine elektrophysiologische Messanordnung gemäß der vorliegenden Erfindung, bei welchen eine jeweilige Apertur durch Wandbereiche gebildet werden, die Mantelflächen unterschiedlicher geometrischer Körper entsprechen.
- **Fig. 8**: zeigt eine elektrophysiologische Messanordnung gemäß einer Ausführungsform der vorliegenden Erfindung, welche nach Art einer so genannten Patchpipette ausgebildet ist.
- **Fig. 9A - 9E**: zeigen in schematischer und geschnittener Seitenansicht verschiedene Aspekte des Einsatzes der erfindungsgemäßen elektrophysiologischen Messanordnung.
- **Fig. 10 - 12**: zeigen in schematischer und geschnittener Seitenansicht Details bei der Anlagerung eines biologischen Objekt an einer jeweiligen Ausführungsform der elektrophysiologischen Messanordnung.

### DETAILBESCHREIBUNG DER AUSFÜHRUNGSFORMEN

Nachfolgend werden Ausführungsformen der vorliegenden Erfindung beschrieben. Sämtliche Ausführungsformen der Erfindung und auch ihre technischen Merkmale und Eigenschaften können einzeln isoliert und wahlfrei zusammengestellt miteinander beliebig und ohne Einschränkung kombiniert werden.

Strukturell und/oder funktionell gleiche, ähnliche oder gleich wirkende Merkmale oder Elemente werden nachfolgend im Zusammenhang mit den Figuren mit denselben Bezugszeichen bezeichnet. Nicht in jedem Fall ihres Auftretens wird eine detaillierte Beschreibung dieser Merkmale oder Elemente wiederholt.

Zunächst wird auf Zeichnungen im Allgemeinen Bezug genommen.

In der Elektrophysiologie wird unter anderem die Patch-Clamp-Technologie angewandt, um z.B. Ionenkanalanalysen für die Medikamententestung durchzuführen. Beim manuellem Patch-Clamp-Verfahren und dessen Verfeinerungen können - z.B. an Einzelzellen - elektrische Ströme und Spannungen gemessen werden, die - z.B. von Ionenkanälen - in den Membranen biologischer Zellen hervorgerufen werden.

Auf Grund der wachsenden Bedeutung elektrophysiologischer Untersuchungen und des personellen und zeitlichen Aufwandes bei deren Durchführung ist eine große Nachfrage nach automatisierten elektrophysiologischen Messtechniken und insbesondere nach planaren Patch-Clamp- und weiteren automatisierten Patch-Clamp- oder APC-Systemen entstanden.

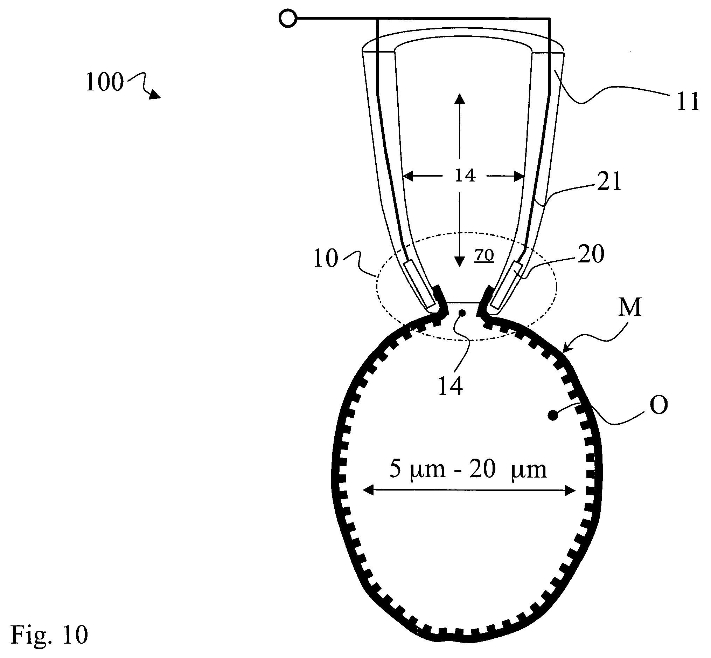

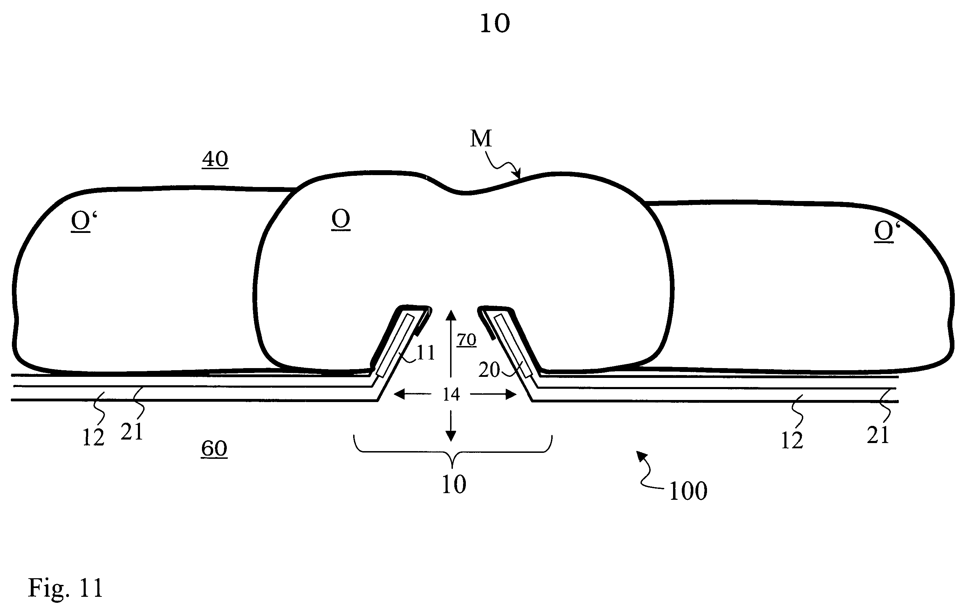

Die Funktionsweisen beim manuellen Patch-Clamp-Verfahren und bei APC-Systemen gegeben sind grundlegend gleich. Problematisch ist bei beiden Systemtypen die Notwendigkeit der Ausbildung eines hochohmigen Abdichtwiderstandes zwischen der dem Messobjekt O, z.B. einer Zelle, und der Messanordnung 100. Man spricht von der Notwendigkeit eines so genannten Gigaseals, also eines Abdichtwiderstands im Gigaohmbereich. Damit wird die elektrische Isolierung - z.B. des Zellinneren zum Zelläußeren - z. B. durch eine Patchpipette beschrieben, wie dies z.B. in Fig. 10 gezeigt ist. oder bei einer flächigen Zellanordnung, wie bei einem APC-Schema gemäß Fig. 11.

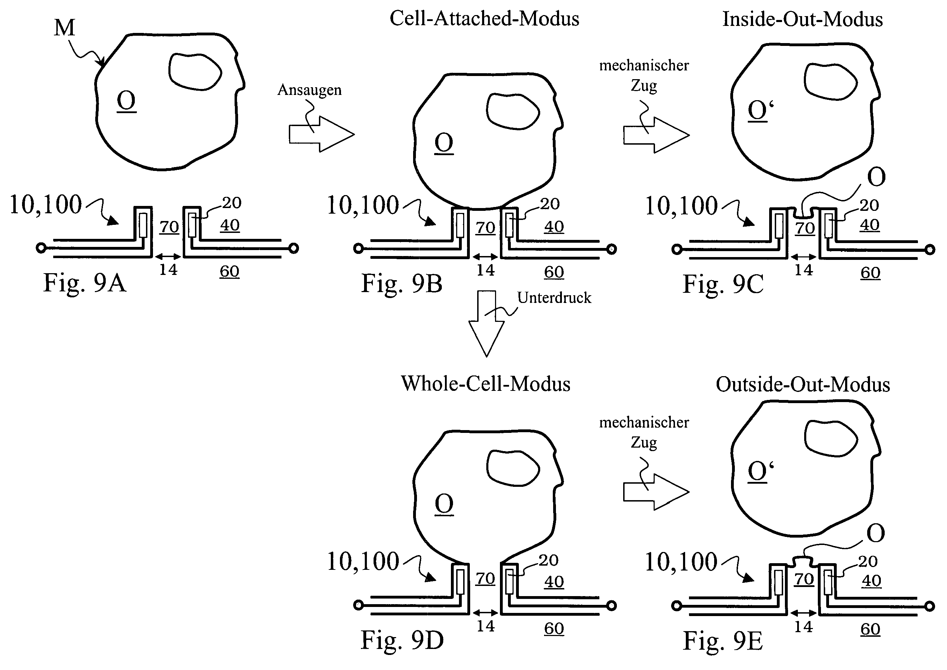

Mittels einer Patchpipette wird eine Zelle als biologisches Objekt O ggf. angesaugt. Ein kleiner, begrenzter Teil der Zellmembran, der als Patch bezeichnet wird, wird dabei mit einem leichten Unterdruck eingesogen. Dadurch wird bei der Cell-Attached-Messung gemäß Fig. 9B die Messfläche bzw. bei einer Whole-Cell-Messung gemäß Fig. 9D das Zellinnere im Mega- bis Gigaohmbereich zum Zelläußeren hin elektrisch abgedichtet. Die Cell-Attached-Konfiguration ermöglicht eine Strommessung auch an einzelnen Ionenkanälen in der Zellmembran.

Die Gigasealrate sowie der Abdichtwiderstand sind beim manuellen Patch-Clamp sowie bei den APC-Systemen ein Maß für die Qualität der möglichen Ionenkanalmessungen. Bis heute ist eine 100 %-ige Gigasealrate nicht möglich.

Die Ausbildung des Gigaseals hängt von vielen Faktoren ab, die einer aktiven Beeinflussung bisher nicht zugänglich sind. Es fehlt also eine echte Steuerung des Gigaseals im Messprozess.

Beim manuellen Patch-Clamp-Verfahren kann nur mit frisch hergestellten Glaspipetten eine ausreichend hohe Gigasealrate erreicht werden. Bei der Entwicklung von Chips für APC-Systeme muss auf eine Reduktion der Oberflächenrauhigkeit und auf eine Vermeidung scharfer Kanten geachtet werden. Weiter kann eine sorgfältige Kombination der intra- und extrazellulären Puffer zu einer Verbesserung des Gigaseals führen. Dies bietet jedoch durch die Notwendigkeit, physiologische Puffer verwenden zu müssen, nur begrenzte Möglichkeiten. Alle Verbesserungen müssen zudem vor dem eigentlichen Experiment bedacht werden.

Bei automatischen Messsystemen, insbesondere für Zellnetzwerke oder Zellkulturen, können in der adhärenten Kultur spontan und unerwünscht Zellen O einen Gigaseal ausbilden. Ein Gigaseal ist jedoch ein zeitlich begrenzter Prozess, an dessen Ende der Gigaseal abreißt und dadurch eine nachfolgende Neuausbildung eines Seals und damit eine erneute Messung verhindert werden. Ein erneutes Verwenden einer Pipette und deren Apertur 14 ist dann nicht mehr möglich.

Somit ist insbesondere für eine Automatisierung eine Steuerung des Gigaseals notwendig. Dies ist bisher nicht möglich.

Die vorliegende Erfindung beruht auf der Erkenntnis, dass negative oder positive Ladungen an oder in der Innenwand 11i einer Messapertur 14, z.B. auch einer Patchpipette, die Ausbildung eines Gigaseals unterstützen und den Gigaseal selbst verbessern können. Durch eine gezielte, ortsaufgelöste Erhöhung der Ladungsdichte - mittels positiver bzw. negativer Ladungen - an oder in der Innenwand 11i der Messapertur 14 werden die Prozesse zur Etablierung oder zur Unterdrückung eines Gigaseals beeinflussbar, wobei ggf. auch elektrokinetische Effekte unter der Anwendung von Wechselfeldern zum Tragen kommen können.

Die Kontrolle der Ladungsdichte an oder in der Pipettenwand 11 wird erfindungsgemäß durch die Integration einer leitenden, zur Innenseite der Messapertur 14 isolierten Steuerelektrode 20, z.B. in Form einer Schicht 20, in die Messaperturwand 11 im Zusammenwirken mit dem Anlegen bestimmter Spannungen und einer Gegenelektrode 30, 50 erreicht.

Eine Ansteuerung dieser Strukturen erfolgt über eine regelbare Spannungsquelle. Diese leitende Steuerelektrode 20 kann symmetrisch zur Messapertur 14 sein. Die Gegenelektrode kann zum Beispiel die Messelektrode 30, 50 des Mess- oder Patch-Clamp-Systems sein, die über eine leitende Flüssigkeit 40, 60 in der Messapertur 14 verbunden ist oder eine eigens dafür vorgesehene Elektrode.

Der Aufbau kann weitestgehend einem Zylinderkondensator entsprechen. Besonders vorteilhaft ist eine Ausführung, bei der die Isolationsschicht zwischen der Elektrode 20 oder leitenden Schicht 20 und der Messapertur 14 besonders dünn ist. Die Ladungsverschiebungen bewirken eine Ansammlung von nur positiven bzw. nur negativen Ladungen an oder in der Innenseite 10i, 11i der Messapertur 14.

Die Erfindung dient also der gesteuerten Etablierung oder Unterdrückung einer abdichtenden Anlagerung eines biologischen Objekts O, z.B. einer Zelle oder dergleichen, im Sinne eines Gigaseals durch das Erzeugen und Kontrollieren von Ladungsdichten an oder in der Innenwand 10i, 11i der Messapertur 14.

Dies geschieht erfindungsgemäß vorrichtungsmäßig durch die Integration eines isolierten Leiters 20 in die Wand 11 der Messapertur 14.

Die durch die Erfindung ermöglichte vergleichsweise hohe Ladungsdichte, sei diese negativ oder positiv, ist konventionell nicht zu erzielen. Bei der hier vorgestellten Erfindung kann dagegen die Messapertur immer kontrollierbar elektrisch geladen werden.

Die vorliegende Erfindung betrifft demzufolge eine elektrophysiologische Messanordnung 100 sowie ein elektrophysiologisches Messverfahren, bei welchen das abdichtende oder sealende Anlagern eines zu untersuchenden biologischen Objekts O an einen Träger 12 der Messanordnung 100 gesteuert werden kann, indem in einem Wandbereich 11, welcher eine Apertur 14 eines Aperturbereichs 10 bildet, in dessen Innern eingebettet, eine Steuerelektrodenanordnung 20 vorgesehen wird, die derart steuerbar mit einem elektrischen Potential beaufschlagbar ist, dass dadurch zumindest die Innenwand 10i, 11i des Wandbereichs 11, welche der Apertur 14 zugewandt ist, steuerbar - in Stärke, zeitlichem und räumlichen Verlauf und/oder Polarität - mit einer Oberflächenladung ausbildbar ist, um so über Wechselwirkung mit der Membran des zu untersuchenden biologischen Objekts O das abdichtende oder sealende Anlagern steuernd zu unterdrücken oder zu unterstützen.

Durch eine geeignet gewählte Elektrodenkombination könnte auch ein Kreisstrom in einer Pipette oder allgemein im Bereich einer Apertur generiert werden, durch welchen die Anlagerung eines Messobjekts ebenfalls unterdrückbar wäre.

Nun wird im Detail auf die Zeichnungen Bezug genommen.

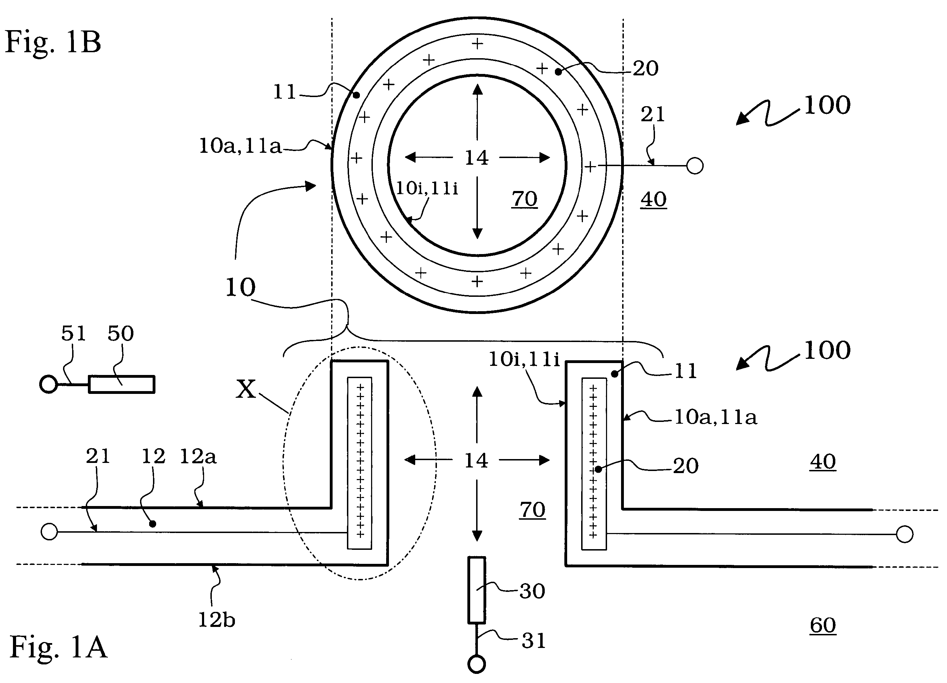

Fig. 1A zeigt in schematischer und geschnittener Seitenansicht eine erste Ausführungsform der erfindungsgemäßen elektrophysiologischen Messanordnung 100.

Ein grundlegendes Element dieser Ausführungsform ist der Träger 12, der auch als Basis 12 oder Substrat 12 bezeichnet werden kann. Dieser Träger 12 unterteilt ein während der Messung vorgesehenes Elektrolytbad 40, 60, 70 in mindestens zwei Kompartimente, wobei das erste Kompartiment 60 der Unterseite 12b des Trägers 12 oder Substrats 12 zugewandt ist und wobei das zweite Kompartiment 40 der Oberseite 12a des Trägers 12 oder Substrats 12 zugewandt ist.

In den Träger 12 ist der so genannte Aperturbereich 10 eingearbeitet. Dieser weist mindestens eine Apertur 14 auf, nämlich z.B. nach Art eines Durchgangslochs, welches den Träger 12 in seiner Schichtdickenrichtung, nämlich in der Richtung von der Oberseite 12a zur Unterseite 12b, lokal vollständig durchdringt. Im Bereich der Apertur 14 ist ebenfalls ein Teil 70 des Elektrolytbads 40, 60, 70 vorgesehen.

Insgesamt gesehen besteht also zwischen der Oberseite 12a und der Unterseite 12b über die Apertur 14 des Aperturbereichs 10 eine fluidmechanische Verbindung und entsprechend über die gegebenenfalls vorgesehene Leitfähigkeit des Elektrolytbads, häufig handelt es sich dabei in der Anwendung um eine physiologische Lösung, auch eine elektrische Verbindung.

Bei der Ausführungsform gemäß Fig. 1A ist im oberseitigen Kompartiment 40 eine mit einer Leitung 51 angeschlossene Gegenelektrode 50 zu einer auf der Unterseite 12b des Trägers 12 vorgesehenen Messelektrode 30 angeordnet. Die Messelektrode 30 befindet sich also im unterseitigen Kompartiment 60 des Elektrolytbades 40, 60, 70 und ist mit einer Leitung 31 angeschlossen. Die Leitungen 51 und 31 zur Gegenelektrode 50 bzw. zur Messelektrode 30 sind an sich isoliert und führen zu einem entsprechenden Steuer- und Messkreis, der hier nicht dargestellt ist.

Im Substrat 12 verläuft - ebenfalls vom Steuer- und Messkreis kommend - eine Steuerleitung 21, die in der Steuerelektrodenanordnung 20 mündet. Diese Steuerelektrodenanordnung 20 ist im Wandbereich 11 in dessen Innerem ausgebildet und somit gegenüber dem Elektrolytbad 40, 60, 70 und auch gegenüber der Messelektrode 30 und der Gegenelektrode 50 elektrisch isoliert.

Alternativ könnte die Steuerelektrodenanordnung 20 nicht wie ein Stab, sondern z.B. nach Art eines Kegels ausgebildet sein, so dass seine Form die Ladungsträgerdichte definiert - in diesem Fall wäre ggf. nur eine einzige Elektrode ausreichend, die jedoch angepasst modifiziert ist. Außerdem sind zusätzlich oder alternativ geteilte Elektroden für die Steuerelektrodenanordnung 20 denkbar, insbesondere auch im Zusammenhang mit einem Wechselspannungsbetrieb.

Der Wandbereich 11 als solcher bildet eine nach Art einer Mantelfläche verlaufende geschlossene Wand mit einer Innenseite 10i, 11i oder Innenwand 10i, 11i und einer Außenseite 10a, 11a oder Außenwand 10a, 11a. Auf diese Art und Weise wird eine Apertur 14 des Aperturbereichs 10 der erfindungsgemäßen elektrophysiologischen Messanordnung 100 gebildet, wobei die Innenseite oder die Innenwand 10i,11i des Wandbereichs 11 der Apertur 14 zugewandt, die Außenwand oder Außenseite 10a,11a des Wandbereichs 11 dagegen der Apertur 14 abgewandt, jedoch dem Kompartiment 40 des Elektrolytbades 40, 60, 70 zugewandt ist.

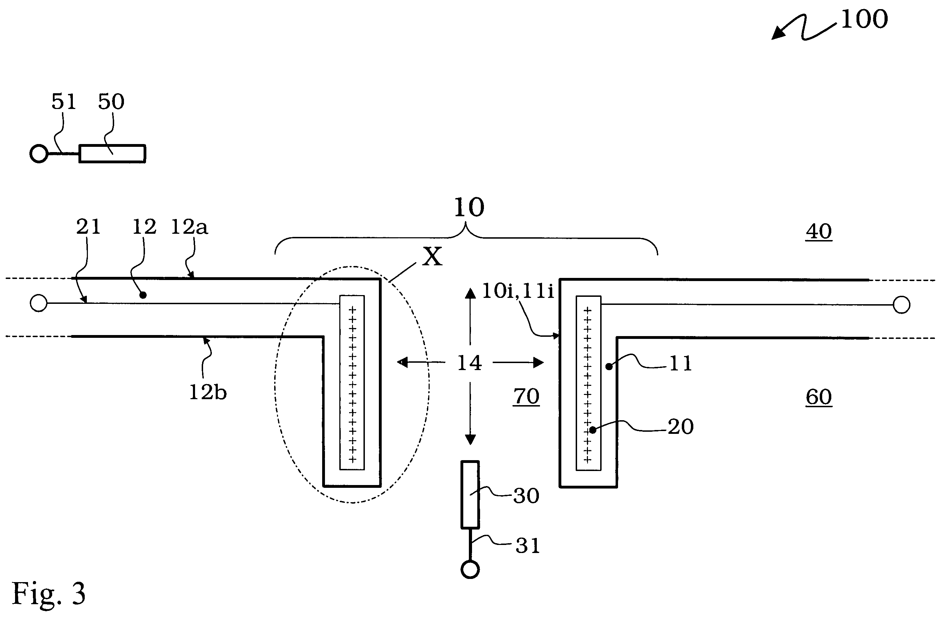

Bei der Ausführungsform der Fig. 1A erhebt sich der Wandbereich 11 ausschließlich oberhalb der Oberseite 12a des Trägers oder Substrats 12. Auf der Unterseite 12b des Substrats oder Trägers 12 ist der Aperturbereich 10 quasi planar ausgebildet. Eine derartige Struktur ist jedoch nicht zwingend und die Fig. 3 bis 7 zeigen diesbezüglich abgewandelte Ausführungsformen, die später im Detail beschrieben werden.

Wie oben erwähnt wurde, ist der Wandbereich 11, welcher die Apertur 14 bildet, nach Art einer Mantelfläche eines geometrischen Körpers ausgebildet. Gemäß Fig. 1B kann in Zusammenschau mit der Fig. 1A diese Mantelfläche von einem senkrechten Kreiszylinder als grundlegender Form stammen.

In Fig. 1A und 1B ist die mit der Leitung 21 angeschlossene Steuerelektrodenanordnung 20 mit positiven elektrischen Ladungen beaufschlagt. Dies ist jedoch nur ein Beispiel. Je nach Situation und zu untersuchendem Objekt kann ein elektrisches Potential an die Steuerelektrodenanordnung 20 angelegt werden, so dass geeignete Wechselwirkungen ausgebildet werden.

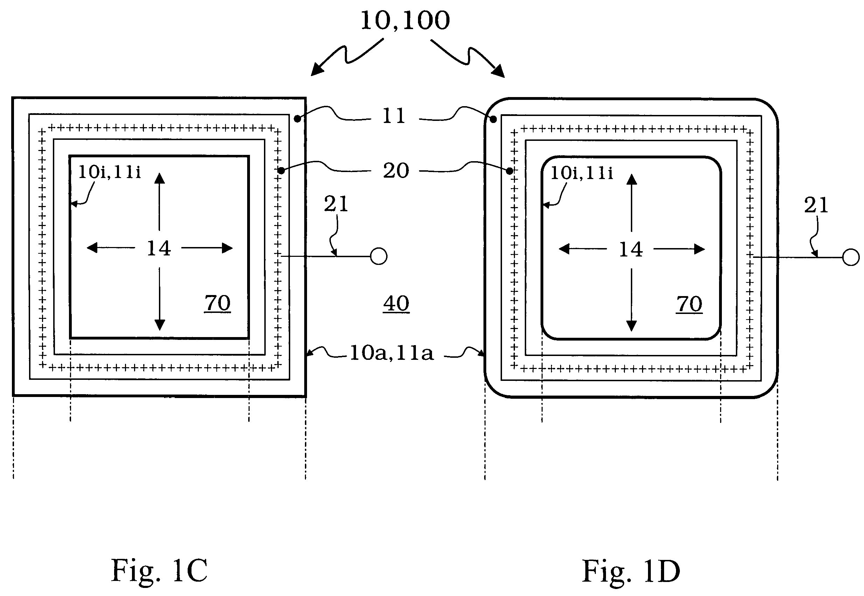

Die Ausbildung als Mantelfläche eines senkrechten Kreiszylinders als grundlegender Form ist nicht zwingend. Die Fig. 1C und 1D zeigen anstelle einer Zylinderform in schematischer Draufsicht als Grundfläche ein Quadrat, so dass sich räumlich eine senkrechtes quadratisches Prisma ergibt bzw. ein Prisma mit einer Grundfläche nach Art eines Ovals, quasi eines Quadrats oder Rechtecks mit abgerundeten Ecken, wie dies in Fig. 1D dargestellt ist.

Es sind grundsätzlich beliebige Grundflächenformen denkbar. Jedoch bieten sich auf Grund der oben beschriebenen Umstände, gemäß welchen Oberflächenrauhigkeiten und scharfe Kanten vermieden werden sollten, gerade Formen mit abgerundeten Strukturen an, also zum Beispiel die Ausgestaltung gemäß der Fig. 1B mit einem senkrechten Kreiszylinder als geometrischer Grundform.

Die die Ladung der Steuerelektrodenanordnung betreffenden Aspekte sind von der Wahl der Form der Mantelfläche im Wesentlichen unabhängig.

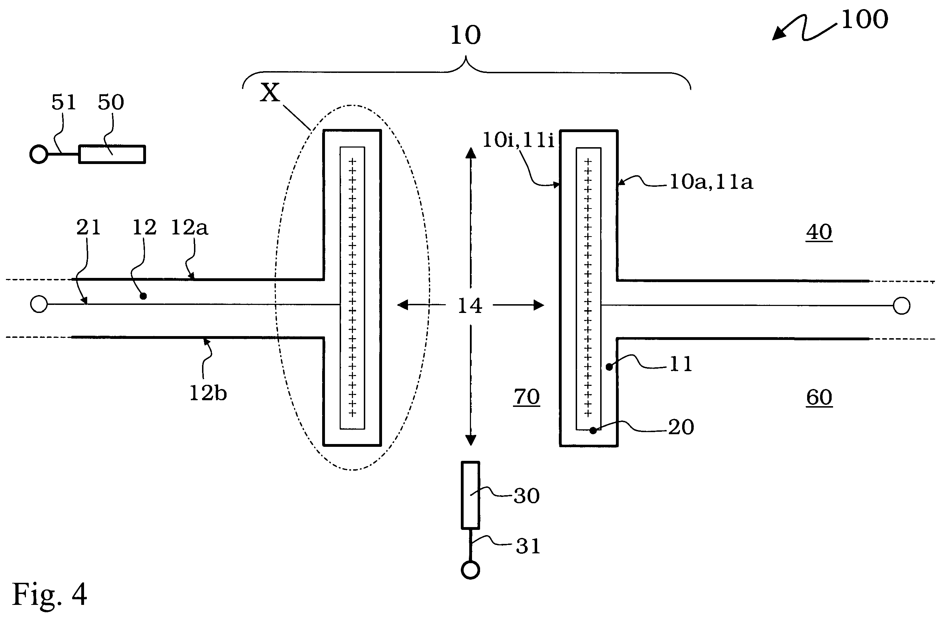

Die Fig. 3 und 4 zeigen analog zu Fig. 1A und ebenfalls in schematischer und geschnittener Seitenansicht andere Ausführungsformen der erfindungsgemäßen Messanordnung, bei welchen ein Unterschied dahingehend besteht, dass nämlich gemäß der Fig. 3 der Wandbereich 11 für die Apertur 14 bündig mit der Oberseite 12a des Substrats 12 abschließt und ausschließlich über die Unterseite 12b des Substrats 12 hinaus hervorsteht, so dass insgesamt eine Art Einstülpung von der Oberseite 12a nach innen zum Kompartiment 60 für die Apertur 14 entsteht.

Bei der Fig. 4 erhebt sich ein Teil des Wandbereichs 11 der Apertur 14 von der Oberseite 12a des Substrats 12 aus in das Kompartiment 40, andererseits jedoch auch ein Teil des Wandbereichs 11 von der Unterseite 12b des Substrats 12 aus in das Kompartiment 60 hinein.

Die Höhen über der Oberseite 12a und über der Unterseite 12b, um welche sich der Wandbereich 11 für die Apertur 14 jeweils erhebt, können identisch sein. Dies ist aber nicht zwingend. In Fig. 4 sind sie unterschiedlich gestaltet.

Bei den Ausführungsformen der Fig. 1A sowie 3 und 4 ist der Querschnitt oder Durchmesser entlang der Erstreckungsrichtung des Wandbereichs 11 senkrecht zur Oberseite 12a bzw. senkrecht zur Unterseite 12b des Substrats 12 konstant in seinem Verlauf.

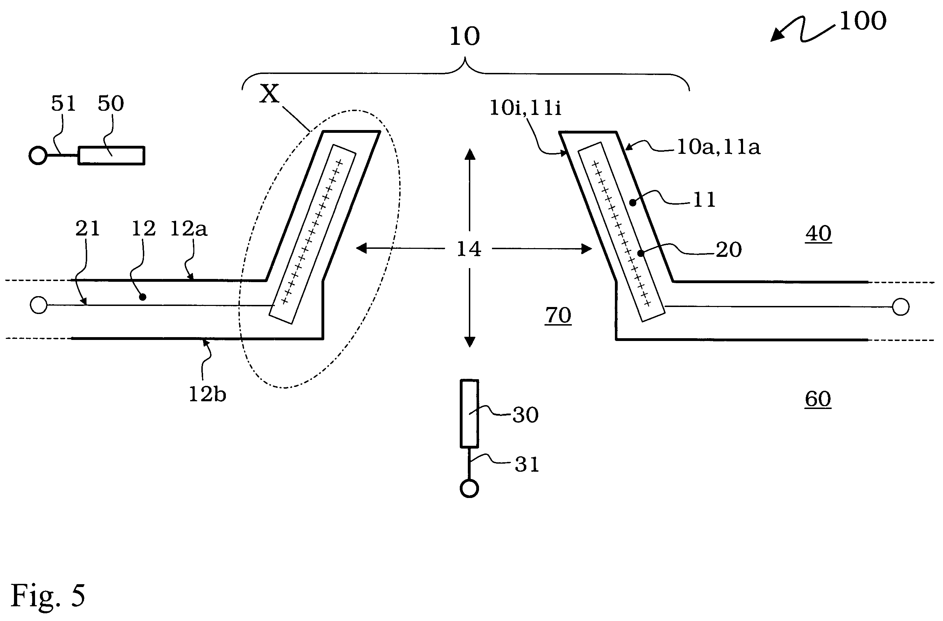

Auch dies ist nicht zwingend. Es können sich verjüngende oder erweiternde Querschnittsverläufe vorgesehen werden. Dies ist in der Abfolge der Fig. 5 bis 7 dargestellt, wobei die Fig. 5 eine Ausführungsform der erfindungsgemäßen Messanordnung zeigt, welche der Ausführungsform der Fig. 1 entspricht, jedoch mit einem Querschnittsverlauf für die Apertur 14, der sich mit zunehmendem Abstand von der Oberseite 12a verengt.

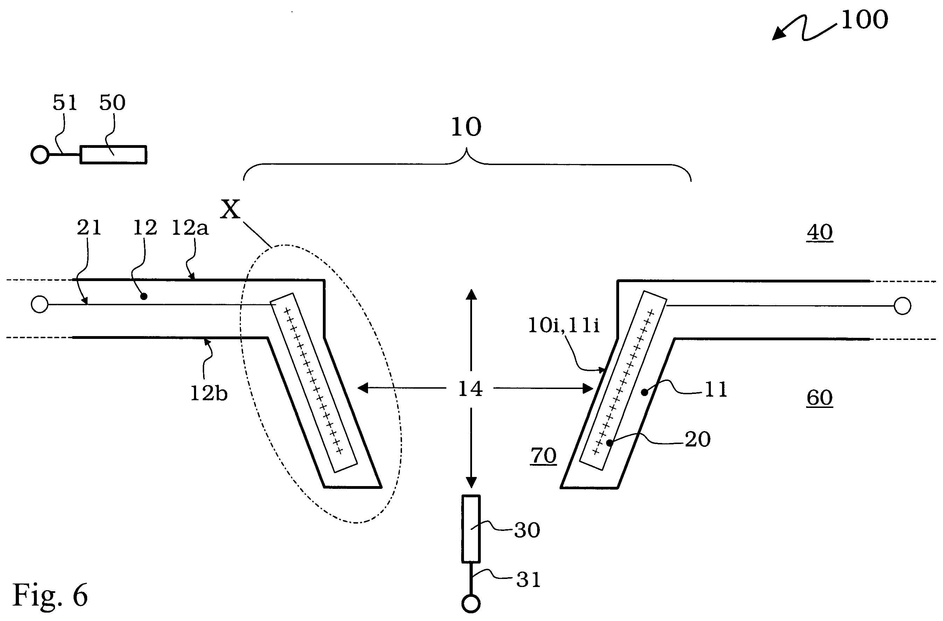

Bei der Ausführungsform der Fig. 6 ist dagegen analog dazu und im Vergleich zur Ausführungsform der Fig. 3 die Apertur 14 so ausgebildet, dass sich der Querschnittsverlauf mit Abstand von der Unterseite 12b des Substrats 12 verjüngt.

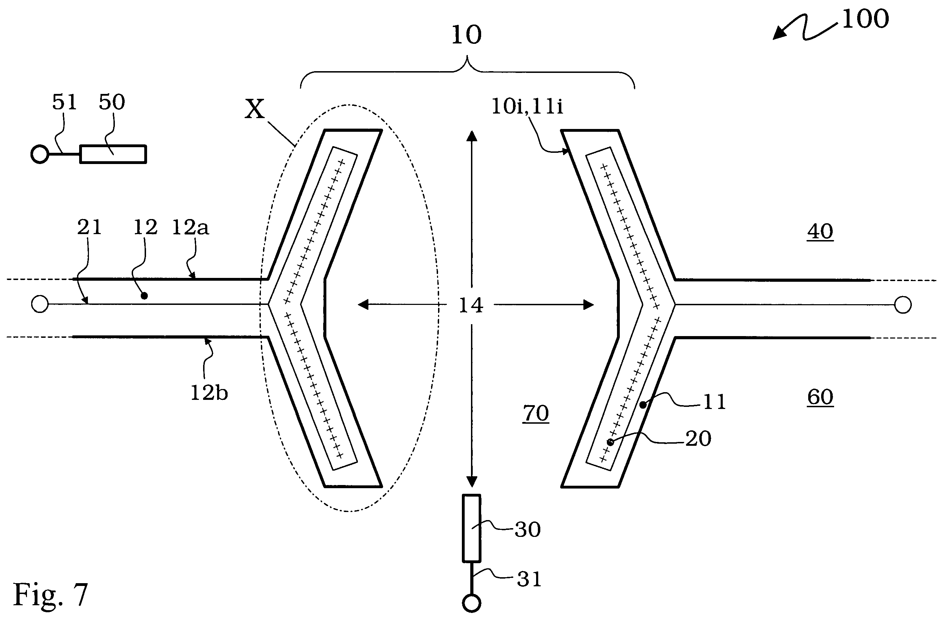

In Kombination der Ausführungsformen der Fig. 5 und 6 und in analoger Sichtweise zur Ausführungsform der Fig. 4 zeigt die Fig. 7 eine Apertur 14, bei welcher auf der Höhe des Substrats 12 der Durchmesser der Apertur 14 maximal ist und sich mit zunehmendem Abstand sowohl von der Oberseite 12a des Substrats 12 als auch von der Unterseite 12b des Substrats 12 aus verjüngt.

Bei der Ausführungsform der Fig. 1A ist die Messelektrode 30 mit dem Anschluss oder Leitung 31 stark benachbart und teilweise in den Aperturbereich 10 mit der Apertur 14 eingeführt ausgebildet. Die Position der Messelektrode 30 kann variiert sein, sie kann zum Beispiel stärker in den Elektrolytbereich 70 in der Apertur 14 hineingeführt sein oder weiter davon entfernt werden.

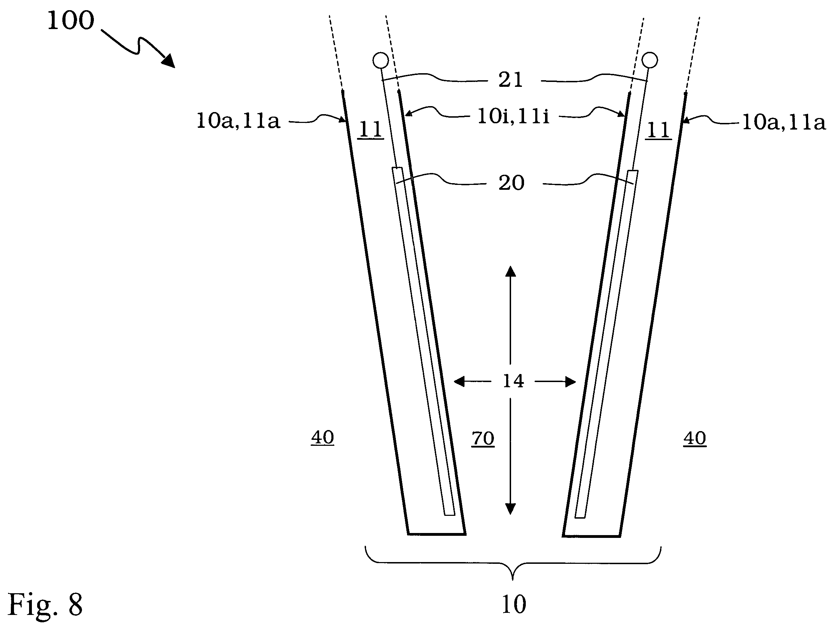

Fig. 8 zeigt in schematischer und geschnittener Seitenansicht eine Ausführungsform der erfindungsgemäßen elektrophysiologischen Messanordnung 100, bei welcher der Aperturbereich 10 von einem Wandbereich 11 gebildet, welcher insgesamt nach Art einer Patchpipette ausgebildet ist.

Auch hier liegen ein Innenwandbereich 10i, 11i, welcher der Apertur 14 zugewandt ist, sowie ein Außenwandbereich 10a, 11a vor, wobei der Außenwandbereich 10a, 11a in der Anwendung einem außen liegenden Elektrolytkompartiment 40 des Bades 40, 60, 70 zugewandt ist und wobei der Innenwandbereich 10i, 11i dem in der Apertur 14 vorgesehenen Elektrolytkompartiment 70 des Bades 40, 60, 70 zugewandt ist. Im Inneren des Wandbereichs 11 und somit vom Elektrolytbad 40, 60, 70 elektrisch isoliert ist wieder eine mittels einer Leitung 21 angeschlossene Steuerelektrodenanordnung 20 ausgebildet, welche in der Lage ist, über eine Beaufschlagung mit einem elektrischen Potential eine Oberflächenladung zumindest auf der Innenwand 10i, 11i des Wandbereichs 11 zu erzeugen, um dadurch eine abdichtende oder sealende Anlagerung eines zu untersuchenden biologischen Objekts O zu verhindern oder zu unterstützen.

Auch hier sind der Vorgang und die Wirkung des Ladens grundsätzlich von der Form der Apertur 14 getrennt zu betrachten.

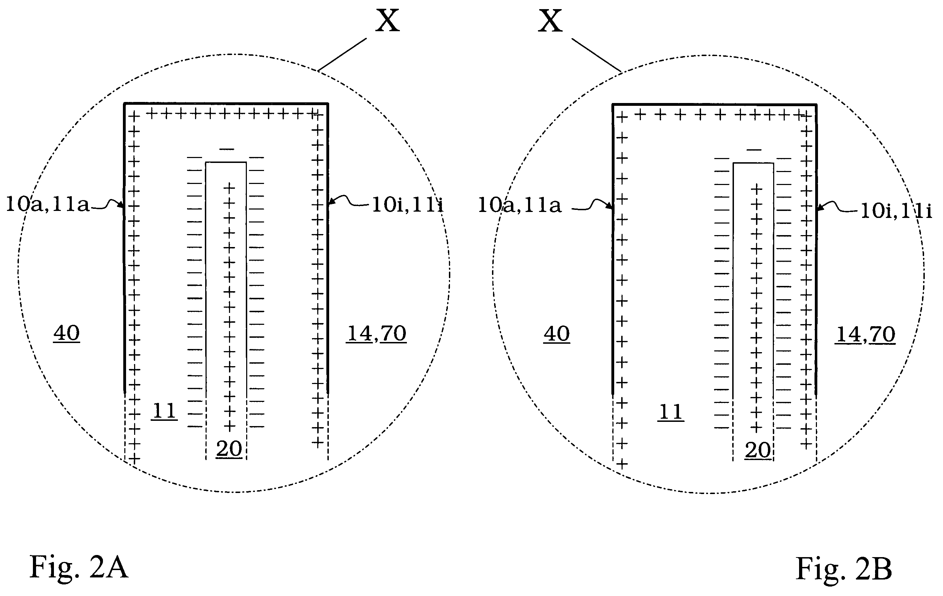

Die Fig. 2A und 2B zeigen in größerem Detail den Ausschnitt X aus der Fig. 1A, nämlich das Induzieren einer - hier positiven - Oberflächenladung auf der Innenseite 10i, 11i und der Außenseite 10a, 11a durch Beaufschlagen der Steuerelektrodenanordnung 20 mit einer - hier positiven - elektrischen Ladung.

Bei der Fig. 2A ist die Steuerelektrodenanordnung 20 in Bezug auf die Schichtstärke des Wandbereichs 11 symmetrisch ausgebildet und angeordnet. Folglich entstehen auf der Innenwand 10i, 11i und auf der Außenwand 10a, 10a gleich starke positive Ladungsdichten. Bei der Ausführungsform gemäß Fig. 2B ist die Steuerelektrodenanordnung 20 dagegen näher zur Innenwand 10i, 11i oder Innenseite 10i, 11i des Wandbereichs 11 his ausgebildet und angeordnet, so dass auf Grund des vergrößerten Abstands zur Außenwand 10a, 11a dort eine geringere positive Ladungsdichte entsteht als auf der Innenwand 10i, 11i.

Die Fig. 9A bis 9E zeigen in schematischer und geschnittener Seitenansicht verschiedene Messprinzipien, die bei der erfindungsgemäßen elektrophysiologischen Messanordnung 100 und dem entsprechenden erfindungsgemäßen elektrophysiologischen Messverfahren zum Tragen kommen können.

Ausgehend von der in Fig. 9A dargestellten Situation, bei welcher durch ein leichtes Ansaugen des Elektrolytbades 40, 60, 70 durch die Messapertur 14, also mit einem Sog vom Kompartiment 40 über das Kompartiment 70 in der Apertur 14 zum Kompartiment 60 hin, wird ein biologisches Objekt O, in diesem Fall zum Beispiel eine Zelle, angesaugt und an die Apertur 14 angenähert wird.

Der Mechanismus des Annäherns kann auch auf eine andere manipulative Art und Weise erfolgen, zum Beispiel mittels einer separaten Pipette, einer Laserpinzette oder dergleichen.

Über einen leichten Unterdruck wird gemäß Fig. 9B die Zelle ohne jegliche Zerstörung und komplett an die Messapertur 14 angelagert. Wird in diesem Zustand eine Messung durchgeführt, so spricht man vom so genannten Cell-Attached-Modus.

Durch einen mechanischen Zug kann, ausgehend von der in Fig. 9B beschriebenen Situation ein Membranfleck aus der Membran des biologischen Objekts O herausgerissen werden, so dass der herausgerissene Membranfleck, ein so genannter Patch, im abgedichteten oder gesealten Zustand in der Messapertur 14 verbleibt und das eigentlich Messobjekt O bildet. Dabei zeigt durch das Herausreißen die vorher im Inneren angeordnete Seite des Membranflecks nach außen, also zum Kompartiment 40 hin. Aus diesem Grund heißt dieser Messmodus auch Inside-Out-Modus.

Andererseits kann, ausgehend von der in Fig. 9B dargestellten Situation durch einen erneuten Unterdruck, nämlich z.B. nach Art eines Druckstoßes die Zelle O insgesamt geöffnet werden, so dass ein Übergang stattfindet vom so genannten Cell-Attached-Modus zum Whole-Cell-Modus, bei dem die Messelektrode 30 direkten Zugriff auf das gesamte Zellinnere hat. Das heißt, das Zellinnere ist zu den Kompartimenten 70 und 60 hin geöffnet, gegenüber dem Kompartiment 40 jedoch im Wesentlichen isoliert.

Von der Situation gemäß der Fig. 9D, nämlich dem Whole-Cell-Modus kann durch mechanischen Zug erreicht werden, dass wiederum ein Membranfragment aus der Membran des biologischen Objekts herausgerissen wird. Da vorher der Whole-Cell-Modus vorlag, besteht eine gewisse Wahrscheinlichkeit dafür, dass dabei der herausgerissene Membranfleck derart in der Messapertur 14 verbleibt, dass er als eigentliches Messobjekt O dienen kann und dabei gemäß Fig. 9E die Außenseite der Zellmembran oder der Membran des biologischen Objekts auch außen verbleibt. In diesem Fall spricht man vom Outside-Out-Modus der Messanordnung 100.

Die Fig. 10 zeigt noch einmal im Detail die geometrische Situation, die bei einem abgedichteten oder sealenden Anlagern eines zu vermessenen biologischen Objekts O zwischen dessen Membran M und der Messapertur 14 und insbesondere der Innenwand 10i, 11i des Wandbereichs 11 vorliegt. Bei der Fig. 10 handelt es sich erneut um einen Whole-Cell-Modus, bei welchem die gesamte Zelle O mit ihrem Inneren zur Messelektrode 30 und zu den Elektrolytkompartimenten 60 und 70 hin geöffnet ist. Diese Ausführungsform der erfindungsgemäßen elektrophysiolgischen Messanordnung 100 ist hier nach Art einer Patchpipette ausgestaltet.

Erfindungsgemäß wird der Abdichtwiderstand zwischen der Zellmembran M des zu vermessenden biologischen Objekts O und der Innenwand 10i, 11i des Wandbereichs 11 der Messapertur 14 dadurch verbessert, dass eine zur Oberflächenladung der Zellmembran M, hier also der Ladung der Außenseite der Zellmembran M entgegengesetzte Ladung auf der Wandinnenseite 10i ausgebildet wird.

Das bedeutet, dass bei einer negativ geladenen Membran M des biologischen Objekts O die Innenwand 10i, 11i des Wandbereichs 11 der Apertur 14 positiv geladen werden muss, dies erfolgt durch ein positives Aufladen der Steuerelektrode 20 über die Leitung 21.

Ist dagegen die anzulagernde Zellmembran M des biologischen Objekts O positiv geladen, so muss die Wandinnenseite 10i, 11i des Wandbereichs 11 der Apertur 14 negativ geladen werden, was eine negative Aufladung der Steuerelektrodenanordnung 20 über die Leitung 21 voraussetzt.

Sollen eine Anlagerung und somit ein Seal verhindert werden, zum Beispiel während einer vorbereitenden Phase für ein Experiment, so muss die Oberflächenladung der Innenwand 10i, 11i des Wandbereichs 11 der Apertur 14 zur Ladung an der Oberfläche der Membran M des biologischen Objekts O gleichnamig aufgeladen werden.

Erfindungsgemäß ist es somit möglich, den verschiedenen Situationen an unterschiedlichen Oberflächen von Membranen, seien dies Zellmembran, Membranen von Organellen oder Membranen von künstlichen Objekten, Rechnung getragen werden. Dies war bisher so nicht möglich und bietet eine Möglichkeit, ein Anlagern und einen Seal gesteuert zu verhindern oder zu fördern bzw., ist ein Seal erst einmal hergestellt, zu stabilisieren.

Bei der Ausführungsform gemäß Fig. 11 ist das Substrat quasi von einer Schicht von Zelle O' belegt. Über die mechanische und/oder elektrische Wechselwirkung unter Einsatz der erfindungsgemäß in der Aperturwand 11 vorgesehenen Steuerelektrodenanordnung 20 und deren gesteuertes Laden ist eine einzelne Zelle O des Ensembles von Zellen O' als Messobjekt im Whole-Cell-Modus analog zur Situation gemäß Fig. 9D bei verbesserter abdichtender oder sealender Anlagerung einer elektrophysiologischen Messung zugänglich.

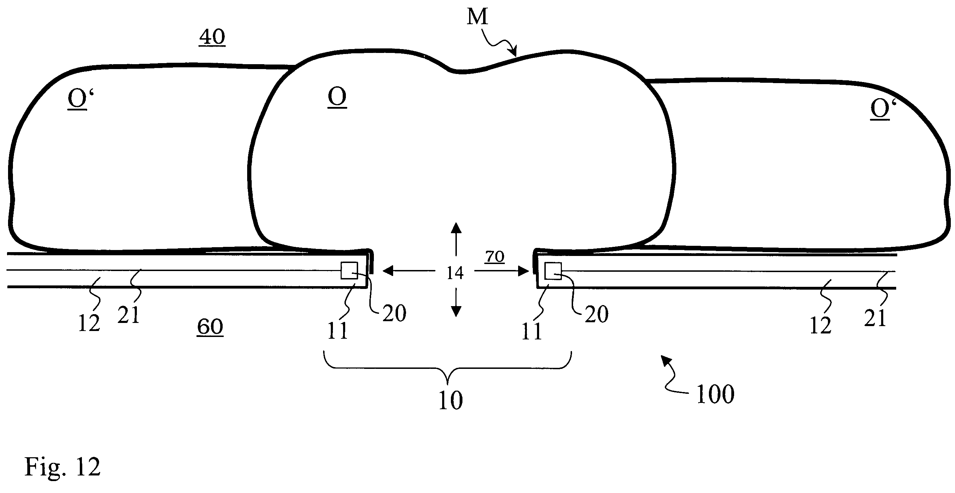

Fig. 12 zeigt eine Anordnung der erfindungsgemäßen Messanordnung 100, bei der der eine Apertur 14 bildende Wandbereich 11 von einem Rand oder Randbereich gebildet wird, so dass die Apertur 14 quasi als planares Loch in dem zu Grunde liegenden Substrat 12 ausgebildet ist und dabei die Steuerelektrodenanordnung 20 den Randbereich mit einer entsprechenden Oberflächenladung beaufschlagt, um das Anlagern und Sealen fördernd oder hemmend zu beeinflussen.

### Bezugszeichenliste

- 10: Aperturbereich
- 10a: Außenwand, Außenwandbereich, Außenseite des Aperturbereichs 10
- 10i: Innenwand, Innenwandbereich, Innenseite des Aperturbereichs 10
- 11: Wandbereich, Wand
- 11i: Innenwand, Innenwandbereich, Innenseite des Wandbereichs 11
- 11a: Außenwand, Außenwandbereich, Außenseite des Wandbereichs 11
- 12: Substrat, Träger, Basis, Grundsubstrat
- 12a: Oberseite, Vorderseite
- 12b: Unterseite, Rückseite
- 14: Apertur, Messapertur
- 20: Steuerelektrodenanordnung, Ladungselektrodenanordnung, Steuerelektrode, Ladungselektrode
- 21: Leitung, Steuerleitung
- 30: Messelektrode, Messelektrodenanordnung
- 31: Messleitung, Leitung
- 40: Elektrolytkompartiment, Elektrolytbad
- 50: Gegenelektrode, Gegenelektrodenanordnung
- 51: Leitung
- 40: Elektrolytkompartiment, Elektrolytbad
- 60: Elektrolytkompartiment, Elektrolytbad
- 70: Elektrolytkompartiment, Elektrolytbad
- 100: elektrophysiologische Messanordnung

- M: Membran des biologischen Objekts O
- O: biologisches Objekt, Zelle, Liposom, Vesikel, Mizelle, Oozyt, Messobjekt
- O': biologisches Objekt, Zelle, Liposom, Vesikel, Mizelle, Oozyt

## Patentansprüche

1. Träger (12) für eine elektrophysiologische Messanordnung (100),
mit einem Aperturbereich (10) zum gesteuerten abdichtenden Anlagern eines biologischen Objekts (O),
wobei der Aperturbereich (10) mit mindestens einer Apertur (14) ausgebildet ist sowie mit einen Wandbereich (11), welcher die Apertur (14) bildend umgibt,
**dadurch gekennzeichnet,**
**dass** der Wandbereich (11) eingebettet in seinem Inneren eine Steuerelektrodenanordnung (20) aufweist und
**dass** die Steuerelektrodenanordnung (20) derart steuerbar mit einem elektrischen Potential beaufschlagbar ist, dass dadurch zumindest eine Innenwand (11i) des Wandbereichs (10), welche der Apertur (14) zugewandt ist, steuerbar derart mit einer Oberflächenladung ausbildbar ist, dass dadurch das abdichtende Anlagern eines biologischen Objekts (O) am Aperturbereich (10) steuerbar ist.

2. Träger (12) nach Anspruch 1,
bei welcher der Aperturbereich (10) im Bereich des Trägers (12), der eine Oberseite (12a) und eine Unterseite (12b) aufweist, ausgebildet ist und
bei welcher ein jeweiliger eine Apertur (14) bildender Wandbereich (11) in Bezug auf die Oberseite (12a) und/oder in Bezug auf die Unterseite (12b) des Trägers (12) teilweise oder vollständig hervorsteht.

3. Träger (12) nach einem der vorangehenden Ansprüche,
bei welcher ein eine Apertur (14) bildender Wandbereich (11) nach Art einer Mantelfläche oder als Kombination von Mantelflächen ausgebildet ist, insbesondere jeweils auf der Grundlage eines Mantels eines Zylinders, eines Prismas, eines Kegelstumpfs und/oder eines Pyramidenstumpfs.

4. Träger (12) nach einem der vorangehenden Ansprüche,
bei welcher ein eine Apertur (14) bildender Wandbereich (11) aus einem Material aus der Gruppe von Materialien ausgebildet ist, welche Glas, Quarzglas, Silizium und Kohlenstoff aufweist.

5. Träger (12) nach einem der vorangehenden Ansprüche,
bei welcher die Steuerelektrodenanordnung (20) ein oder mehrere und im eine Apertur (14) bildenden Wandbereich (11) integrierte Elektrodenelemente aufweist, insbesondere in Form von Ringen, Streifen oder dergleichen.

6. Träger (12) nach einem der vorangehenden Ansprüche,
bei welcher die Steuerelektrodenanordnung (20) in Bezug auf die Wandstärke des die Apertur (14) bildenden Wandbereichs (11) asymmetrisch und insbesondere näher zur Innenwand (11i), welcher der Apertur (14) zugewandt ist, hin angeordnet ist.

7. Träger (12) nach einem der vorangehenden Ansprüche,
bei welcher die Steuerelektrodenanordnung (20) aus einem Material aus der Gruppe von Materialien ausgebildet ist die metallische Materialien, Gold, Tantal, Platin, Gold-Tantal-Platin, dotiertes, insbesondere hochdotiertes Polysilizium, Indiumzinnoxid, elektrisch leitfähige organische Materialien aufweist.

8. Träger (12) nach einem der vorangehenden Ansprüche,
bei welcher der Durchmesser der Apertur (14) und insbesondere der Innendurchmesser des die Apertur (14) bildenden Wandbereichs (11) einen Wert im Bereich von etwa 1 µm bis etwa 50 µm aufweist und/oder
bei welcher der eine Apertur (14) bildende Wandbereich (11) über der Oberseite (12a) oder der Unterseite (12b) des Trägers (12) eine Höhe oder Tiefe im Bereich von etwa 0 µm bis etwa 20 µm aufweist.

9. Elektrophysiologische Messanordnung (100) mit einem Träger (12) nach einem der vorangehenden Ansprüche,
bei welcher eine Messelektrode (30) im Bereich (70) oder innerhalb der Apertur (14) oder in einem Bereich (60) auf der Unterseite (12b) des Trägers (12) vorgesehen ist und
bei welcher eine Gegenelektrode (50) außerhalb der Apertur (14) und im Bereich (40) auf der Oberseite (12a) des Trägers (12) vorgesehen ist.

10. Elektrophysiologisches Messverfahren unter Verwendung einer elektrophysiologischen Messanordnung (100) nach Anspruch 9,
bei welchem ein zu vermessendes biologisches Objekt (O) an der Apertur (14) angelagert und dann elektrophysiologisch vermessen wird und
bei welchem zum Steuern eines abdichtenden Anlagerns eines zu vermessenden biologischen Objekts (O) an der Apertur (14) des Aperturbereichs (10) die Innenwand (11i) des die Apertur (14) bildenden Wandbereichs (11) gesteuert mit einer entsprechend geeigneten Oberflächenladung ausgebildet wird,
wobei zum Verhindern oder Unterstützen einer abdichtenden oder sealenden Anlagerung entsprechend dem Typ des zu vermessenden biologischen Objekts (O) eine negative oder positive Oberflächenladung und zum Unterstützen einer sealenden Anlagerung eine positive oder negative Oberflächenladung ausgebildet werden,
wobei das Verfahren die Schritte aufweist:
(1) Bereitstellen adhärierter oder suspendierter biologischer Objekte (O) auf dem Träger (12),
(2) An- und Einsaugen der Membran (M) eines im Bereich der Apertur (14) befindlichen biologischen Objekts (O) mittels Unterdruck über die Apertur (14),
(3) Laden der Elektrodenelemente der Steuerelektrodenanordnung (20) und
(4) dadurch laterale Attraktion der an- und eingesaugten Abschnitte der Membran (M) und abdichtendes Anlagern der an- und eingesaugten Abschnitte der Membran (M) an der Innenwand (11i) der Apertur (14),
(5) Durchführen einer elektrophysiologischen Messung am im Bereich der Apertur (14) befindlichen biologischen Objekts (O).

## Claims

1. A support (12) for an electrophysiological measuring arrangement (100),
with an aperture region (10) for controllable sealing depositing of a biological object (O),
wherein the aperture region (10) is formed with at least one aperture (14) as well as with a wall region (11), which surrounds the aperture (14) to form it,
**characterized in**
**that** the wall region (11) comprises embedded into its interior a control electrode arrangement (20) and
**that** an electric potential may be applied to the control electrode arrangement (20) in a controllable manner such that by this at least an inner wall (11i) of the wall region (10), which faces the aperture (14) can be controllably formed with a surface charge such that by this the sealing depositing of a biological object (O) at the aperture region (10) is controllable.

2. The support (12) according to claim 1,
wherein the aperture region (10) is formed in a region of the support (12), which comprises an upper side (12a) and a lower side (12b), and
wherein a respective wall region (11) forming an aperture (14) partially or completely protrudes with respect to the upper side (12a) and/or with respect to the lower side (12b) of the support (12).

3. The support (12) according to one of the preceding claims,
wherein a wall region (11) forming an aperture (14) is formed in the manner of a lateral surface or as combination of lateral surfaces, in particular on the basis of a lateral surface of a cylinder, a prism, a truncated cone and/or a truncated pyramid, respectively.

4. The support (12) according to one of the preceding claims,
wherein a wall region (11) forming an aperture (14) is formed from a material from the groups of materials, which comprises glass, quartz glass, silicone, and carbon.

5. The support (12) according to one of the preceding claims,
wherein the control electrode arrangement (20) comprises one or more electrode elements integrated within a wall region (11) forming an aperture (14), in particular in the form of circles, stripes or the like.

6. The support (12) according to one of the preceding claims,
wherein the control electrode arrangement (20) is asymmetrically with respect to the wall thickness of the wall region (11) forming the aperture (14) and is in particular arranged closer to the inner wall (11i), which faces the aperture (14).

7. The support (12) according to one of the preceding claims,
wherein the control electrode arrangement (20) is formed from a material of the group of materials, which comprises metallic materials, gold, tantalum, platinum, gold-tantalum-platinum, doped, in particular highly doped, polysilicon, indium tin oxide, electrically conductive organic materials.

8. The support (12) according to one of the preceding claims,
wherein the diameter of the aperture (14) and in particular the inner diameter of the wall region (11) forming the aperture (14) has a value in the region of about 1 *µ*m to about 50 *µ*m and/or
wherein the wall region (11) forming an aperture (14) has a height or depth with respect to the upper side (12a) or the lower side (12b) of the support (12) in the range of about 0 *µ*m to about 20 *µ*m.

9. An electrophysiological measuring arrangement (100) with a support (12) according to one of the preceding claims,
wherein a measurement electrode (30) is provided in the region (70) or within the aperture (14) or in a region (60) at the lower side (12b) of the support (12) and
wherein a counter-electrode (50) is provided outside of the aperture (14) and in the region (40) at the upper side (12a) of the support (12).

10. An electrophysiological measuring method, using an electrophysiological measuring arrangement (100) according to claim 9,
wherein a biological object (O) to be measured is deposited at the aperture (14) and then electrophysiologically measured,
wherein for controlling a sealing depositing of a biological object (O) to be measured on the aperture (14) of the aperture region (10) the inner wall (11i) of the wall region (11) forming the aperture (14) is formed in a controlled manner with an accordingly suited surface charge,
wherein for preventing or supporting of sealing depositing according to the type of the biological object (O) to be measured a negative or positive surface charge and for supporting of a sealing depositing a positive or negative surface charge is formed,
wherein the method comprises the steps:
(1) providing adhering or suspended biological objects (O) on the support (12),
(2) aspirating and sucking in of the membrane (M) of an biological object (0) located in the region of the aperture (14) via the aperture (14) by an underpressure,
(3) charging of the electrode elements of the control electrode arrangement (20), and
(4) due to this, lateral attraction of the aspirated and sucked in parts of the membrane (M) and sealing depositing of the aspirated and sucked in parts of the membrane (M) to the inner wall (11i) of the aperture (14),
(5) performing an electrophysiological measurement of the biological object (O) located in the region of the aperture (14).

## Revendications

1. Support (12) pour un dispositif de mesure électrophysiologique (100),
avec une zone d'ouverture (10) pour l'application étanche commandée d'un objet biologique (O),
étant précisé que la zone d'ouverture (10) est pourvue d'au moins une ouverture (14) et d'une zone de paroi (11) qui entoure l'ouverture (14) en lui donnant sa forme,
**caractérisé**
**en ce que** la zone de paroi (11) comporte, encastré à l'intérieur, un dispositif d'électrode de commande (20), et
**en ce que** le dispositif d'électrode de commande (20) est apte à être sollicité avec un potentiel électrique, de manière commandée, de telle sorte qu'au moins une paroi intérieure (11i), tournée vers l'ouverture (14), de ladite zone de paroi (10) soit pourvue de manière commandée d'une charge superficielle de telle sorte que l'application étanche d'un objet biologique (0) contre la zone d'ouverture (10) soit ainsi apte à être commandée.

2. Support (12) selon la revendication 1, avec lequel la zone d'ouverture (10) est formée dans la zone du support (12), qui présente une face supérieure (12a) et une face inférieure (12b), et
avec lequel une zone de paroi (11) qui forme une ouverture (14) dépasse en partie ou complètement de la face supérieure (12a) et/ou de la face inférieure (12b) du support (12).

3. Support (12) selon l'une des revendications précédentes, avec lequel une zone de paroi (11) qui forme une ouverture (14) est formée à la manière d'une surface latérale ou comme une combinaison de surfaces latérales, en particulier sur la base d'une surface latérale d'un cylindre, d'un prisme, d'un cône tronqué et/ou d'une pyramide tronquée.

4. Support (12) selon l'une des revendications précédentes, avec lequel une zone de paroi (11) qui forme une ouverture (14) se compose d'un matériau du groupe de matériaux comprenant le verre, le verre de quartz, le silicium et le carbone.

5. Support (12) selon l'une des revendications précédentes, avec lequel le dispositif d'électrode de commande (20) comporte un ou plusieurs éléments d'électrode intégrés dans la zone de paroi (11) qui forme une ouverture (14), en particulier sous la forme d'anneaux, de bandes ou autres.

6. Support (12) selon l'une des revendications précédentes, avec lequel le dispositif d'électrode de commande (20) est asymétrique par rapport à l'épaisseur de paroi de la zone de paroi (11) qui forme l'ouverture (14), et est disposé en particulier plus près de la paroi intérieure (11i) qui est tournée vers l'ouverture (14).

7. Support (12) selon l'une des revendications précédentes, avec lequel le dispositif d'électrode de commande (20) se compose d'un matériau du groupe de matériaux qui comprend des matériaux métalliques, l'or, le tantale, le platine, or-tantale-platine, le polysilicium dopé, en particulier fortement dopé, l'oxyde indium-étain, des matériaux organiques conducteurs d'électricité.

8. Support (12) selon l'une des revendications précédentes, avec lequel le diamètre de l'ouverture (14) et en particulier le diamètre intérieur de la zone de paroi (11) qui forme l'ouverture (14) présente une valeur située dans la plage d'environ 1 µm à environ 50 µm et/ou
avec lequel la zone de paroi (11) qui forme une ouverture (14) présente au-dessus de la face supérieure (12a) ou de la face inférieure (12b) du support (12) une hauteur ou une profondeur située dans la plage d'environ 0 µm à environ 20 µm.

9. Dispositif de mesure électrophysiologique (100) avec un support (12) selon l'une des revendications précédentes, avec lequel une électrode de mesure (30) est prévue dans la zone (70) ou à l'intérieur de l'ouverture (14) ou dans une zone (60) située sous la face inférieure (12b) du support (12), et
avec lequel une contre-électrode (50) est prévue à l'extérieur de l'ouverture (14) et dans la zone (40) située sur la face supérieure (12a) du support (12).

10. Procédé de mesure électrophysiologique à l'aide d'un dispositif de mesure électrophysiologique (100) selon la revendication 9,
avec lequel un objet biologique à mesurer (O) est appliqué contre l'ouverture (14) et est ensuite soumis à une mesure électrophysiologique, et
avec lequel pour la commande d'une application étanche d'un objet biologique à mesurer (0) contre l'ouverture (14) de la zone d'ouverture (10), la paroi intérieure (11i) de la zone de paroi (11) qui forme l'ouverture (14) est pourvue de manière commandée d'une charge superficielle appropriée correspondante,
étant précisé que pour empêcher ou favoriser une application étanche ou colmatée selon le type de l'objet biologique à mesurer (0) une charge superficielle négative ou positive et pour favoriser une application colmatée une charge superficielle positive ou négative sont formées,
étant précisé que le procédé comporte les étapes suivantes :
(1) fourniture d'objets biologiques (O) collés ou suspendus, sur le support (12),
(2) succion et aspiration de la membrane (M) d'un objet biologique (O) qui se trouve dans la zone de l'ouverture (14), à l'aide d'une dépression par l'intermédiaire de l'ouverture (14),
(3) chargement des éléments d'électrode du dispositif d'électrode de commande (20), et
(4) de ce fait attraction latérale desdites sections aspirées de la membrane (M) et application étanche de celles-ci contre la paroi intérieure (11i) de l'ouverture (14),
(5) exécution d'une mesure électrophysiologique sur l'objet biologique (0) qui se trouve dans la zone de l'ouverture (14).
